# EUROPÄISCHE PATENTSCHRIFT

(11) **EP 3 334 479 B1**
(45) Veröffentlichungstag und Bekanntmachung des Hinweises auf die Patenterteilung: **13.11.2019**
(21) Anmeldenummer: 16757558.8
(22) Anmeldetag: 10.08.2016
(51) Int. Cl.: A61M 1/36, A61M 1/16, A61M 1/34

(54) **BLUTBEHANDLUNGSVORRICHTUNG**
BLOOD TREATMENT DEVICE
DISPOSITIF DE TRAITEMENT DE SANG

(30) Priorität: 11.08.2015 DE 102015010417
(43) Veröffentlichungstag der Anmeldung: 20.06.2018
(73) Patentinhaber: Fresenius Medical Care Deutschland GmbH, 61352 Bad Homburg (DE)
(72) Erfinder: KREUZBERG, Ursula, 51371 Leverkusen (DE); SCHOLZ, Cäcilia, 65824 Schwalbach (DE); STUARD, Stefano, 61348 Bad Homburg (DE); WEHMEYER, Wolfgang, 72076 Tübingen (DE)
(74) Vertreter: Herrmann, Uwe
(86) Internationale Anmeldenummer: PCT/EP2016/001382
(87) Internationale Veröffentlichungsnummer: WO 2017/025199

(56) Entgegenhaltungen:
- EP-A1- 2 583 700
- WO-A1-2013/182287
- A AMORE ET AL: "Use of alkaline rinsing solution to prevent hypersensitivity reactions during hemodialysis: data from a multicentre retrospective analysis", JOURNAL OF NEPHROLOGY : JN, Bd. 12, Nr. 6, 1. November 1999 (1999-11-01), Seiten 383-389, XP055317356, IT ISSN: 1121-8428
- JEAN-LOUIS RENAUX ET AL: "Activation of the kallikrein-kinin system in hemodialysis: Role of membrane electronegativity, blood dilution, and pH", KIDNEY INTERNATIONAL, Bd. 55, Nr. 3, 1. März 1999 (1999-03-01), Seiten 1097-1103, XP055316914, LONDON, GB ISSN: 0085-2538, DOI: 10.1046/j.1523-1755.1999.0550031097.x in der Anmeldung erwähnt
- ROSANNA COPPO ET AL: "Bradykinin and nitric oxide generation by dialysis membranes can be blunted by alkaline rinsing solutions", KIDNEY INTERNATIONAL, Bd. 58, Nr. 2, 1. August 2000 (2000-08-01) , Seiten 881-888, XP055318389, LONDON, GB ISSN: 0085-2538, DOI: 10.1046/j.1523-1755.2000.00238.x in der Anmeldung erwähnt

## Beschreibung

Die vorliegende Erfindung betrifft eine Blutbehandlungsvorrichtung mit wenigstens einem extrakorporalen Kreislauf und mit wenigstens einem Dialysatkreislauf sowie mit zumindest einem Dialysator, der wenigstens eine blutseitige Kammer und wenigstens eine von der blutseitigen Kammer durch eine oder mehrere Membranen getrennte dialysatseitige Kammer aufweist, wobei die blutseitige Kammer mit dem extrakorporalen Kreislauf und die dialysatseitige Kammer mit dem Dialysatkreislauf in Fluidverbindung steht. Des Weiteren weist die Blutbehandlungsvorrichtung eine Zubereitungseinheit zur Online-Herstellung einer Lösung auf.

Derartige Blutbehandlungsvorrichtungen sind beispielsweise in Form von Hämodialysegeräten aus dem Stand der Technik bekannt. Bei diesen bekannten Geräten dient die Zubereitungseinheit zur Online-Herstellung des Dialysates und ggf. des Substituates. Dabei ist unter der "Online-Herstellung" zu verstehen, dass die Lösung, d.h. also beispielsweise das Dialysat oder Substituat im Gerät selbst hergestellt wird und nicht z.B. in Containern oder dergleichen an das Dialysegerät verbracht wird.

EP2583700 A1 offenbart z.B. ein Hämodialysegerät mit einer Zubereitungseinheit zur online-Herstellung einer Lösung und ein Verfahren zum Starten einer Hämodialyse.

Bei diversen Membranoberflächen des Dialysators kann es beim Erstkontakt des Blutes des behandelten Patienten zu einer so genannten Kontaktphasenaktivierung kommen. Diese Kontaktphasenaktivierung wird einerseits durch das Auffalten von Proteinen des Blutes durch einen pH-Wert unterhalb des physiologischen Niveaus von 7,4 und andererseits durch Bindungspunkte auf der künstlichen Oberfläche, wie beispielsweise durch Ladungen ausgelöst.

Den Einfluss des pH-Wertes des Blutes auf die Bio-Kompatibilität des Gesamtsystems wird beispielsweise in J. Renaux et al.: Activation of the kallikrein-kinin system in hemodialysis: Role of membrane electronegativity, blood dilution and pH (Kidney International, Vol. 55 (1999, S. 1097-1103) sowie in R. Coppo et al.: Importance of the bradykinin-nitric oxide synthase system in the hypersensitivity reactions of chronic hemodialysis patients (Nephrol Dial Transplant (2000) 15: 1288-1290) beschrieben.

Der vorliegenden Erfindung liegt die Aufgabe zugrunde, eine Blutbehandlungsvorrichtung der eingangs genannten Art dahingehend weiterzubilden, dass die Wahrscheinlichkeit für das Auftreten der Kontaktphasenaktivierung gegenüber bekannten Blutbehandlungsvorrichtungen verringert ist.

Diese Aufgabe wird durch eine Blutbehandlungsvorrichtung mit den Merkmalen des Anspruchs 1 gelöst.

Dabei ist vorgesehen, dass die Blutbehandlungsvorrichtung erste Mittel zur Durchführung eines Priming-Modus und zweite Mittel zur Durchführung eines initialen Behandlungsmodus aufweist, wobei die ersten Mittel derart ausgebildet sind, dass diese die Zubereitungseinheit so ansteuern, dass eine Spüllösung mit einem pH-Wert von ≥ 7,3 und vorzugsweise von ≥ 7,4 hergestellt wird, und dass der extrakorporale Kreislauf mit dieser Spüllösung gefüllt wird. Des Weiteren sind die zweiten Mittel derart ausgebildet, dass diese die Zubereitungseinheit so ansteuern, dass Dialysat mit einem pH-Wert von ≥ 7,3 und vorzugsweise von ≥ 7,4 hergestellt wird und dass in einer ersten Phase der Behandlung in einem Behandlungsmodus das Dialysat mit dem genannten pH-Wert den Dialysator durchfließt und/oder als Substituat dem extrakorporalen Blutkreislauf zugeführt wird. Des Weiteren weist die Blutbehandlungsvorrichtung wenigstens eine Steuer- oder Regelungseinheit auf, die die ersten Mittel vor den zweiten Mitteln betreibt, sodass zunächst das Priming und dann die Anfangsphase der eigentlichen Blutbehandlung durchgeführt werden.

Der Erfindung liegt somit der Gedanke zugrunde, für das Primen einen pH-Wert nahe dem physiologischen pH-Wert von Blut einzustellen und auch noch während der Anfangsphase der Behandlung über eine gewisse Zeit den erfindungsgemäßen pH-Wert beizubehalten.

Der pH-Wert des Dialysats oder Substituats kann, muss jedoch nicht identisch zu dem pH-Wert der Priminglösung sein.

Erst nach Abschluss der Anfangsphase können durch dritte Mittel die Zusammensetzung des Dialysats bzw. des Substituats an die verschriebene Zusammensetzung bzw. Konzentration angepasst werden.

Die vorliegende Erfindung macht sich die Erkenntnis zu Nutze, dass der pH-Wert nicht nur während des Primens sondern auch noch in der Anfangsphase der Blutbehandlung von Bedeutung ist. Während dieser Anfangsphase, in der der pH-Wert in dem erfindungsgemäßen Bereich gehalten wird, kommt es zu einer Belegung der Membran des Dialysators mit Proteinen und Zellen, sodass keine freie künstliche Oberfläche mehr vorliegt, die mit Proteinen des Blutes in Erstkontakt treten können. Die Wahrscheinlichkeit für die Kontaktphasenaktivierung ist somit minimiert.

Bei der Membran handelt es sich vorzugsweise um eine Vielzahl von Hohlfasermembranen, die zu einem Bündel in einem Gehäuse zusammengefasst sind.

Aus dem Stand der Technik ist es zwar bekannt, den extrakorporalen Kreislauf vor der Behandlung einem Priming zu unterziehen, jedoch ist es im Stand der Technik nicht bekannt, dabei den erfindungsgemäßen pH-Wert einzustellen und auch nicht, den erfindungsgemäßen pH-Wert während der Anfangsphase der Blutbehandlung beizubehalten. Des Weiteren ist es keine übliche Praxis, sich über die ärztliche Verschreibung gerade in dieser Phase hinwegzusetzen, so dass das erfindungsgemäße Vorgehen daher eine überraschende Lehre darstellt.

Der historische Stand der Technik ist das Primen mit Kochsalzlösung. Da diese Lösung keinen Puffer enthält, wird der pH-Wert des Blutes in diesem Fall trotz Verdünnung nicht verändert. Insbesondere bei Patienten mit ausgeprägter metabolischer Azidose kann es hierbei zum Auslösen von Kontaktphasenaktivierung kommen, sofern die künstlichen Membranoberflächen hinreichend viele Bindungspunkte anbieten.

Bei bekannten Online-HDF(Hämodiafiltration)-Maschinen wird in der Regel die Zusammenfassung der Substitionslösung, die gleichzeitig auch als Dialysat verwendet wird auch zum Primen verwendet. Der Nachteil dieser Lösung besteht darin, dass der pH-Wert beim Primen nicht unabhängig von der Einstellung zur Korrektur der metabolischen Azidose ist. So kann es beim Andialysieren von neuen Patienten ratsam sein, die Korrektur der metabolischen Azidose über mehrere Behandlungen zu strecken und anfangs mit einer vergleichsweise geringen Bicarbonatkonzentration zu fahren. Die Folge davon ist, ein pH-Wert der Priming-Lösung unterhalb des physiologischen Bereiches, womit eine Kontaktphasenaktivierung einhergehen kann.

Die ersten, zweiten und dritten Mittel können durch ein und dasselbe Bauteil gebildet werden. In diesem Falle besteht somit eine Unterscheidung zwischen den Mitteln nur in dem Betriebsmodus, in dem sie betrieben werden, d.h. die ersten Mittel werden in einem ersten Betriebsmodus (während des Priming), die zweiten Mittel in einem zweiten Betriebsmodus (während der initialen Behandlungsphase) und die dritten Mittel werden in einem dritten Betriebsmodus (während der Behandlungsphase nach Abschluss der initialen Behandlungsphase) betrieben. Die Zubereitungseinheit wird in Abhängigkeit des Betriebsmodus der Mittel betrieben.

Von der Erfindung ist jedoch auch der Fall umfasst, dass alle die Mittel oder wenigstens zwei der Mittel durch unterschiedliche Bauteile gebildet werden, die in den vorgenannten Modi betrieben werden.

Das Mittel kann auch ein Programmcode sein, der derart ausgebildet ist, dass er bestimmte Module, z.B. die Pumpen in unterschiedlichen Phasen unterschiedlich ansteuert. Dies bedeutet, dass im Programmcode für jede Phase andere Werte (Mengen, Konzentrationen) hinterlegt sind, die dann zu unterschiedlichen Mischungsverhältnissen der Konzentrate mit dem Wasser führen.

Bei dem oder den Bauteilen kann es sich beispielsweise um einen Controller, Rechner, Prozessor etc. handeln, der die Zubereitungseinheit ansteuert, so dass diese die gewünschte Lösung je nach Betriebsmodus der Mittel herstellt. Die Steuer- oder Regelungseinheit stellt den Betriebsmodus der Mittel ein, was zu einem entsprechenden Betrieb der Zubereitungseinheit führt. Die Mittel können einen integralen Bestandteil der Steuer- oder Regelungseinheit darstellen oder gegenüber der Steuer- oder Regelungseinheit als gesonderte Elemente ausgeführt sein.

Die ersten und die zweiten Mittel können so ausgebildet sein, dass diese die Zubereitungseinheit in identischer Weise ansteuern, was zur Folge hat, dass die Zusammensetzung der Spüllösung für das Primen und des Dialysates für die Anfangsphase der Behandlung identisch sind.

In einer weiteren Ausgestaltung sind die zweiten Mittel so ausgebildet, dass diese die Zubereitungseinheit derart ansteuern, das Substituat mit einem pH-Wert von ≥ 7,3 und vorzugsweise von ≥ 7,4 hergestellt wird. Dabei kann vorgesehen sein, dass die zweiten Mittel so ausgebildet sind, dass diese die Zubereitungseinheit zur Herstellung des Dialysats und vom Substituat in identischer Weise ansteuern. Dies hat zur Folge, dass die Zusammensetzungen des Dialysats und des Substituats identisch sind.

In einer bevorzugten Ausgestaltung sind die Zusammensetzungen des Dialysats des Substituats und der Spüllösung zumindest bis Abschluss der Anfangsphase der Dialysebehandlung identisch.

In einer weiteren Ausgestaltung der Erfindung ist vorgesehen, dass die zweiten Mittel so ausgeführt sind, dass diese für eine vorbestimmte Zeitspanne oder für eine Dauer betrieben werden, die von zumindest einem Parameter abhängt. Bei dieser vorbestimmten Zeit kann es sich beispielsweise um einen Zeitraum von bis zu 15 Minuten handeln. Bei diesem Wert handelt es sich jedoch nur um einen exemplarischen Wert.

Bei dem Parameter kann es sich beispielsweise um die Menge des über die Membran des Dialysates extrahierten Plasmas oder um einen damit korrelierten Parameter handeln. So ist es beispielsweise denkbar, die Anfangsphase so lange durchzuführen, bis eine festgelegte Menge an extrahiertem Plasma vorliegt.

Auch ist es denkbar, als Parameter, der über die Dauer der Anfangsphase entscheidet, den Ultrafiltrationskoeffizienten der Membran heranzuziehen, um eine Abschätzung über den Aufbau einer Sekundärmembran zu erzielen.

Denkbar ist es somit, dass die Anfangsphase nach Ablauf einer festen Zeit und/oder nach Erhalt einer fixen Menge an über die Membran extrahiertem Plasma endet.

Vorzugsweise sind die ersten Mittel derart ausgebildet, dass im Rahmen des Primens außer dem extrakorporalen Kreislauf auch die dialysatseitige Kammer des Dialysators mit Spüllösung gefüllt wird.

In einer weiteren Ausgestaltung der Erfindung weist die Blutbehandlungsvorrichtung dritte Mittel zur Durchführung eines auf den initialen Behandlungsmodus folgenden Behandlungsmodus auf, wobei die dritten Mittel derart ausgebildet sind, dass diese die Zubereitungseinheit so ansteuern, dass das Dialysat und/oder das Substituat in seiner Zusammensetzung an eine verschriebene Zusammensetzung angeglichen wird oder dieser entsprechen. Mittels der Steuer- oder Regelungseinheit des Gerätes werden die dritten Mittel nach den zweiten Mitteln betrieben. Dies bedeutet, dass im Anschluss an die Anfangsphase das Dialysat und/oder das Substituat an die verschriebene Zusammensetzung bzw. Konzentration angepasst wird, was stufenweise oder auch mittels einer Rampe bzw. stetig erfolgen kann.

In einer bevorzugten Ausgestaltung der Erfindung erfolgt die Einstellung des pH-Wertes der jeweiligen Lösung mittels der Bicarbonatkonzentration der in der Zubereitungseinheit hergestellten Lösung.

Somit kann die Zubereitungseinheit Fördermittel zur Förderung von Bicarbonat und insbesondere eines Bicarbonat-haltigen Konzentrats aufweisen, wobei die Fördermittel so ausgebildet sind, dass diese die Einstellung des pH-Wertes durch die Förderung von Bicarbonat vornehmen.

So lässt sich über die Menge bzw. Konzentration von Bicarbonat ein bestimmter pH-Wert der Spüllösung, des Dialysats bzw. des Substituats einstellen.

In einer Ausgestaltung der Erfindung ist die Zubereitungseinheit so ausgeführt, dass die Förderung von Bicarbonat in Abhängigkeit von einer Säurekonzentration, z.B. der Acetatkonzentration oder der Citratkonzentration der in der Zubereitungseinheit hergestellten Lösung erfolgt. Die Acetatkonzentration kann bekannt sein, z.B. aus einer internen Datentabelle oder auch gemessen werden. In Abhängigkeit von der Acetatkonzentration wird sodann die Bicarbonatkonzentration eingestellt, um einen bestimmten pH-Wert der Lösung zu erhalten.

In einer weiteren Ausgestaltung der Erfindung ist vorgesehen, dass in dem Dialysatkreislauf wenigstens ein Fördermittel zur Förderung von Dialysat angeordnet ist, vorzugsweise eine Pumpe, und dass die Steuer- oder Regelungseinheit so ausgebildet ist, dass das Fördermittel hinsichtlich seiner Förderrate verringert oder ganz angehalten wird, wenn die zweiten Mittel aktiv sind, d.h. während der Anfangsphase der Behandlung.

Durch die erfindungsgemäße Einstellung des pH-Wertes kann es bei sonst unveränderten Parametern zu einer Erhöhung der Geschwindigkeit der Korrektur der metabolischen Azidose im Blut des Patienten kommen. Das Endniveau ist nicht beeinflusst.

Dieser Anfangseffekt wird insbesondere bei den inzidenten Patienten am höchsten sein, da dort der Unterschied zwischen der Bicarbonatkonzentration im Priming-Fluid bzw. während der Anfangsphase der Dialyse zur Verschreibung des Dialysates am Höchsten ist. Der Unterschied kann beispielsweise 5 bis 7 mmol/l betragen. Um nun den Stofftransport von Bicarbonat in das Blut zu begrenzen und damit den zeitlichen Anstieg des pH-Wertes im Blut langsamer zu gestalten ist es denkbar, den Dialysatfluss in der Anfangsphase der Behandlung, d.h. also während die zweiten Mittel aktiv sind zu begrenzen und im Extremfall auf den Wert Null zu setzen. In diesem Fall wird mit einer reinen Hämofiltration begonnen. Eine derartige Vorgehensweise ist aus der DE 10 2012 011 196 A1 bekannt.

In einer weiteren Ausgestaltung der Erfindung ist vorgesehen, dass die ersten bzw. zweiten bzw. dritten Mittel so ausgebildet sind, dass die Spüllösung bzw. dass das Dialysat bzw. das Substituat einen pH-Wert im Bereich zwischen 7,3 bis 7,7, vorzugsweise im Bereich zwischen 7,35 bis 7,7 und besonders bevorzugt im Bereich zwischen 7,35 bis 7,45 aufweist.

Die Zubereitungseinheit kann eine Hauptleitung aufweisen, die mit einem Wasseranschluss, insbesondere für RO-Wasser in Verbindung steht. In diese Hauptleitung können eine oder mehrere Konzentratleitungen münden, die mit einem oder mehreren Konzentratreservoirs in Fluidverbindung stehen. Aus diesen Reservoirs kann durch Pumpen das Konzentrat wie beispielsweise ein saures Konzentrat und ein basisches Konzentrat in die Hauptleitung befördert werden, um auf diese Weise die fertige Lösung (Spüllösung, Dialysat oder Substitutionslösung) herzustellen.

Weitere Einzelheiten und Vorteile der Erfindung werden anhand eines in der Zeichnung dargestellten Ausführungsbeispiels näher erläutert.

Es zeigen:
- Figur 1:: die Abhängigkeit des pH-Wertes der Lösung von der Bicarbonatkonzentration sowie von der Essigsäure- bzw. Acetatkonzentration und
- Figur 2:: ein exemplarisches Ablaufschema zum Betrieb einer Blutbehandlungsvorrichtung gemäß der Erfindung.

In einer bevorzugten Ausgestaltung der Erfindung betrifft das Ausführungsbeispiel ein Hämodialysegerät mit einem extrakorporalen Blutkreislauf, dessen Leitungen über einen venösen und über einen arteriellen Zugang mit dem Patienten verbunden sind bzw. verbunden werden können. Diese Leitungen stehen mit einem Dialysator in Verbindung, der durch eine Vielzahl von Hohlfasern in blutseitige Kammern und in eine dialysatseitige Kammer unterteilt ist. Die dialysatseitige Kammer umgibt die Hohlfasern und steht mit dem Dialysatkreislauf in Verbindung bzw. bildet einen Teil von diesem.

Der Dialysatkreislauf weist eine Zufuhrleitung zum Dialysator auf, die mit der Zubereitungseinheit in Verbindung steht sowie eine Ablaufleitung, die zum Ablauf für das verbrauchte Dialysat führt. In dieser Ablaufleitung befindet sich eine Dialysatpumpe. Im Blutkreislauf befindet sich vorzugsweise in Strömungsrichtung des Blutes stromaufwärts des Dialysators die Blutpumpe.

Des Weiteren kann eine Substituatleitung vorgesehen sein, die von der Zubereitungseinheit in den extrakorporalen Kreislauf mündet und zwar vor und/oder nach dem Dialysator, sodass eine Prädilution oder Postdilution möglich ist.

Die Zubereitungseinheit weist eine Hauptleitung auf, die mit einer Quelle für RO-Wasser in Verbindung steht. Des Weiteren sind Konzentratleitungen vorgesehen, durch die saures Konzentrat einerseits und basisches Konzentrat andererseits mittels entsprechender Förderpumpen in die Hauptleitung gefördert werden. Über einen oder mehrere Leitfähigkeitsmesszellen kann die korrekte Zusammensetzung der auf diese Weise hergestellten Lösung bestimmt werden.

Die in der Zubereitungseinheit hergestellte Lösung wird als Spüllösung, als Dialysat und als Substituat verwendet.

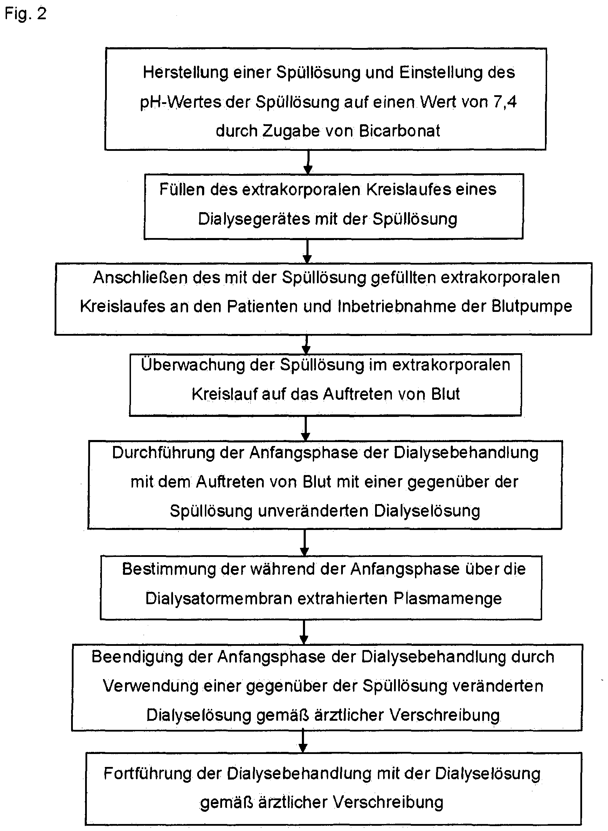

Ein beispielhaftes Ablaufschema zum Betrieb einer erfindungsgemäßen Blutbehandlungsvorrichtung zeigt Figur 2.

Vor der eigentlichen Blutbehandlung erfolgt das Primen des extrakorporalen Kreislaufes mit der Spüllösung. Dabei wird die Zusammensetzung der Priming-Lösung so gesteuert, dass bei Anschluss des Blutkreislaufes sowohl die dialysatseitige Kammer als auch der gesamte extrakorporale Kreislauf mit einer Lösung bzw. mit der Spüllösung gefüllt ist, die einen pH-Wert von ≥ 7,4 hat.

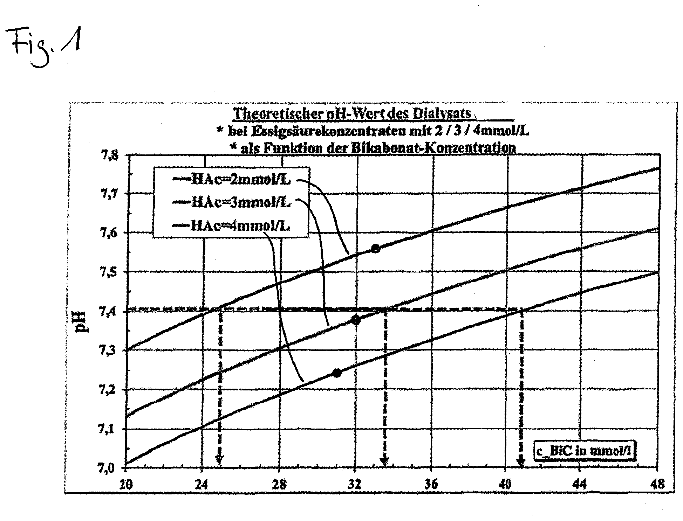

Zur Realisierung dieses Ziels dosiert die Zubereitungseinheit der Blutbehandlungsvorrichtung Bikarbonat in Abhängigkeit der Acetatkonzentration entsprechend einer internen Datentabelle ein. Figur 1 zeigt die entsprechende Korrelation. In dieser Figur sind unterschiedliche Acetat- bzw. Essigsäurekonzentrationen von 2, 3 und 4 mmol/l dargestellt. Auf der Ordinate ist der pH-Wert und auf der Abszisse die Bikarbonatkonzentration angegeben, die in Abhängigkeit von der Acetatkonzentration entsprechend unterschiedlich eingestellt werden muss, um den angestrebten pH-Wert von 7,4 zu erhalten. So ist bei einer Essigsäurekonzentration von 2 mmol/l eine Bikarbonatkonzentration von ca. 25 mmol/l, bei einer Essigsäurekonzentration von 3 mmol/l eine Bikarbonatkonzentration von ca. 33,5 mmol/l und bei einer Essigsäurekonzentration von 4 mmol/l eine Bikarbonatkonzentration von ca. 41 mmol/l erforderlich, um den angestrebten pH-Wert von 7,4 zu erhalten.

Nach dem Primen wird der mit der Spüllösung gefüllte extrakorporale Kreislauf an den Patienten angeschlossen, d.h. an den venösen und an den arteriellen Zugang.

Anschließend wird die Blutpumpe in Betrieb genommen.

Bei der Detektion von Blut im venösen Schenkel des Schlauchsystems des extrakorporalen Kreislaufs wird die Zusammensetzung der Priming-Lösung für das Dialysat bzw. für das Substituat zunächst übernommen, d.h. nicht verändert. Die Dialyse wird nun in dieser Anfangsphase so lange mit einem Dialysat mit physiologischen pH-Wert gefahren, bis von einem Abschluss der Proteinbeladung der Membran ausgegangen werden kann. Wie oben ausgeführt, kann dies entweder nach einer festen Zeit und/oder nach Erhalt einer fixen Menge an über die Membran extrahiertem Plasma der Fall sein. Als Kriterium für den Abschluss der Anfangsphase der Behandlung kann auch der Verlauf des Ultrafiltrationskoeffizienten der Membran herangezogen werden, um eine Abschätzung über den Aufbau einer Sekundärmembran zu erzielen. Es kann eine Tabelle vorgesehen sein, in der für verschiedene Filtertypen entsprechende Abschlusskriterien hinterlegt sein können.

Die Zusammensetzung der Priming-Lösung für das Substituat kann auch in dem pH-Wertbereich ≥ 7,3 bzw. ≥ 7,4 gehalten oder nicht verändert werden.

Ist eine solche Sekundärmembran erzielt, ist die Wahrscheinlichkeit für das Auftreten der Kontaktphasenaktivierung minimiert.

Nach Abschluss dieser Anfangsphase kann z.B. über eine Rampenfunktion die Bikarbonatkonzentration des Dialysats und des Substituats an die verschriebene Konzentration angepasst werden, d.h. gesenkt werden, sodass sich geringere pH-Werte ergeben, als während des Primens und der Anfangsphase der Behandlung.

Wie oben ausgeführt, kann während der Anfangsphase der Behandlung die Dialysatpumpe mit gegenüber dem Normalbetrieb verringerter Rate betrieben werden oder ganz angehalten werden. Dies hat den Vorteil, dass der Stofftransport von Bikarbonat in das Blut begrenzt wird und somit die Geschwindigkeit der Korrektur der metabolischen Azidose herabgesetzt wird.

Die vorliegende Erfindung betrifft die erfindungsgemäße Blutbehandlungsvorrichtung. Ein Verfahren zur Durchführung einer Blutbehandlung mittels der Behandlungsvorrichtung wird auch beschrieben.

Gemäß dem Verfahren wird zunächst eine Spüllösung mit einem pH-Wert von ≥ 7,3 und vorzugsweise von ≥ 7,4 in den extrakorporalen Kreislauf und zusätzlich ggf. in die dialysatseitige Kammer gefüllt und im Anschluss an das Primen die Behandlung ebenfalls mit einem Dialysat durchgeführt, dass einen pH-Wert von ≥ 7,3 und vorzugsweise von ≥ 7,4 aufweist. Diese Anfangsphase der Dialysebehandlung kann für eine bestimmte Zeitspanne oder solange durchgeführt werden, bis eine bestimmte Menge an Blut durch die Membran geströmt ist.

## Patentansprüche

1. Blutbehandlungsvorrichtung mit einem extrakorporalen Kreislauf und mit einem Dialysatkreislauf sowie mit einem Dialysator, der eine blutseitige Kammer und eine von der blutseitigen Kammer durch eine Membran getrennte dialysatseitige Kammer aufweist, wobei die blutseitige Kammer mit dem extrakorporalen Kreislauf und die dialysatseitige Kammer mit dem Dialysatkreislauf in Fluidverbindung steht, sowie mit einer Zubereitungseinheit zur online-Herstellung einer Lösung, **dadurch gekennzeichnet, dass** die Blutbehandlungsvorrichtung erste Mittel zur Durchführung eines Priming-Modus und zweite Mittel zur Durchführung eines initialen Behandlungsmodus aufweist, wobei die ersten Mittel derart ausgebildet sind, dass diese die Zubereitungseinheit derart ansteuern, dass eine Spüllösung mit einem pH-Wert von ≥ 7,3 und vorzugweise von ≥ 7,4 hergestellt wird, und dass der extrakorporale Kreislauf mit dieser Spüllösung gefüllt wird, und dass die zweiten Mittel derart ausgebildet sind, dass diese die Zubereitungseinheit derart ansteuern, dass Dialysat mit einem pH-Wert von ≥ 7,3 und vorzugweise von ≥ 7,4 hergestellt wird, und dass der Dialysatkreislauf mit diesem Dialysat gefüllt wird, wobei die Blutbehandlungsvorrichtung eine Steuer- oder Regelungseinheit aufweist, die die ersten Mittel vor den zweiten Mitteln betreibt, wobei die Zubereitungseinheit Fördermittel zur Förderung von Bicarbonat und insbesondere eines Bicarbonat-haltigen Konzentrats aufweist und die Fördermittel derart ausgebildet sind, dass diese die Einstellung des pH-Wertes mittels der Förderung von Bicarbonat vornehmen, und wobei die Blutbehandlungsvorrichtung dritte Mittel zur Durchführung eines auf den initialen Behandlungsmodus folgenden Behandlungsmodus aufweist, wobei die dritten Mittel derart ausgebildet sind, dass diese die Zubereitungseinheit derart ansteuern, dass das Dialysat in seiner Zusammensetzung an eine verschriebene Zusammensetzung angeglichen wird, wobei die Bicarbonatkonzentration gesenkt wird, und wobei die Steuer- oder Regelungseinheit derart ausgebildet ist, dass die dritten Mittel nach den zweiten Mittel betrieben werden.

2. Blutbehandlungsvorrichtung nach Anspruch 1, **dadurch gekennzeichnet, dass** die ersten und die zweiten Mittel derart ausgebildet sind, dass diese die Zubereitungseinheit in identischer Weise ansteuern, so dass die Zusammensetzungen der Spüllösung und des Dialysats im initialen Behandlungsmodus identisch sind.

3. Blutbehandlungsvorrichtung nach Anspruch 1 oder 2, **dadurch gekennzeichnet, dass** die zweiten oder weitere Mittel derart ausgebildet sind, dass diese die Zubereitungseinheit derart ansteuern, dass Substituat mit einem pH-Wert von ≥ 7,3 und vorzugweise von ≥ 7,4 hergestellt wird, wobei vorzugsweise vorgesehen ist, dass die zweiten Mittel derart ausgebildet sind, dass diese die Zubereitungseinheit zur Herstellung des Dialysats und von Substituat in identischer Weise ansteuern, so dass die Zusammensetzungen des Dialysats und des Substituats identisch sind.

4. Blutbehandlungsvorrichtung nach einem der vorhergehenden Ansprüche, **dadurch gekennzeichnet, dass** die zweiten Mittel derart ausgeführt sind, dass diese für eine vorbestimmte Zeitspanne oder für eine Dauer betrieben werden, die von zumindest einem Parameter abhängt.

5. Blutbehandlungsvorrichtung nach Anspruch 4, **dadurch gekennzeichnet, dass** es sich bei dem Parameter um die Menge des über die Membran des Dialysators extrahierten Plasmas oder einem damit korrelierten Parameter oder um den Ultrafiltrationskoeffizienten der Dialysatormembran handelt.

6. Blutbehandlungsvorrichtung nach einem der vorhergehenden Ansprüche, **dadurch gekennzeichnet, dass** die ersten Mittel derart ausgebildet sind, dass außer dem extrakorporalen Kreislauf auch die dialysatseitige Kammer mit Spüllösung gefüllt wird.

7. Blutbehandlungsvorrichtung nach einem der vorhergehenden Ansprüche, **dadurch gekennzeichnet, dass** die dritten Mittel derart ausgebildet sind, dass diese die Zubereitungseinheit derart ansteuern, dass das Substituat in seiner Zusammensetzung an eine verschriebene Zusammensetzung angeglichen wird.

8. Blutbehandlungsvorrichtung nach einem der vorhergehenden Ansprüche, **dadurch gekennzeichnet, dass** die Zubereitungseinheit derart ausgeführt ist, dass die Förderung von Bicarbonat in Abhängigkeit von der Acetat- oder Citratkonzentration der in der Zubereitungseinheit hergestellten Lösung erfolgt.

9. Blutbehandlungsvorrichtung nach einem der vorhergehenden Ansprüche, **dadurch gekennzeichnet, dass** in dem Dialysatkreislauf ein Fördermittel zur Förderung von Dialysat angeordnet ist und dass die Steuer- oder Regelungseinheit derart ausgebildet ist, dass das Fördermittel hinsichtlich seiner Förderrate verringert oder angehalten wird, wenn die zweiten Mittel aktiv sind.

10. Blutbehandlungsvorrichtung nach einem der vorhergehenden Ansprüche, **dadurch gekennzeichnet, dass** die Mittel derart ausgebildet sind, dass die Spüllösung bzw. das Dialysat bzw. das Substituat einen pH-Wert im Bereich zwischen 7,3 bis 7,7, vorzugsweise im Bereich zwischen 7,35 bis 7,7 und besonders bevorzugt im Bereich zwischen 7,35 bis 7,45 aufweist.

11. Blutbehandlungsvorrichtung nach einem der vorhergehenden Ansprüche, **dadurch gekennzeichnet, dass** die Zubereitungseinheit eine Hauptleitung aufweist, die mit einem Wasseranschluss, insbesondere mit einem RO-Wasseranschluss in Verbindung steht und dass in diese Hauptleitung eine oder mehrere Konzentratleitungen münden, die mit einem oder mehreren Konzentratreservoirs in Fluidverbindung stehen.

## Claims

1. Blood treatment apparatus having an extracorporeal circuit and a dialyzate circuit as well as a dialyzer which comprises a blood-side chamber and a dialyzate-side chamber which is separated from the blood-side chamber by a membrane, wherein the blood-side chamber is in fluid communication with the extracorporeal circuit and the dialyzate chamber is in fluid communication with the dialyzate circuit, and having a preparation unit for the online preparation of a solution, **characterized in that** the blood treatment apparatus comprises first means for carrying out a priming mode, and second means for carrying out an initial treatment mode, wherein the first means are configured such that they control the preparation unit such that a flushing solution having a pH of ≥ 7.3, and preferably of ≥ 7.4, is prepared and such that the extracorporeal circuit is filled with this flushing solution, and the second means are configured such that they control the preparation unit such that dialyzate having a pH of ≥ 7.3, and preferably of ≥ 7.4, is prepared, and such that the dialyzate circuit is filled with this dialyzate, wherein the blood treatment apparatus comprises a control or regulation unit which operates the first means before the second means, wherein the preparation unit comprises conveying means for conveying bicarbonate and in particular a concentrate containing bicarbonate; and **in that** the conveying means are configured such that they carry out the setting of the pH by means of the conveying of bicarbonate, and wherein the blood treatment apparatus comprises third means for carrying out a treatment mode following the initial treatment mode, wherein the third means are configured such that they control the preparation unit such that the dialyzate is matched in its composition to a prescribed composition, wherein the control or regulation unit is configured such that the third means are operated after the second means.

2. Blood treatment apparatus in accordance with claim 1, **characterized in that** the first means and the second means are configured such that they control the preparation unit in an identical manner such that the compositions of the flushing solution and of the dialyzate are identical in the initial treatment mode.

3. Blood treatment apparatus in accordance with claim 1 or 2, **characterized in that** the second means or further means are configured such that they control the preparation unit such that substituate having a pH of ≥ 7.3, and preferably of ≥ 7.4, is prepared, wherein provision is preferably made that the second means are configured such that they control the preparation unit for preparing the dialyzate and of substituate in an identical manner so that the compositions of the dialyzate and of the substituate are identical.

4. Blood treatment apparatus in accordance with one of the preceding claims, **characterized in that** the second means are configured such that they are operated for a predetermined time or for a duration which depends on at least one parameter.

5. Blood treatment apparatus in accordance with claim 4, **characterized in that** the parameter is the quantity of the plasma extracted over the membrane of the dialyzer or a parameter correlated therewith or the ultrafiltration coefficient of the dialyzer membrane.

6. Blood treatment apparatus in accordance with one of the preceding claims, **characterized in that** the first means are configured such that the dialyzate-side chamber is also filled with flushing solution in addition to the extracorporeal circuit.

7. Blood treatment apparatus in accordance with one of the preceding claims, **characterized in that** the third means are configured such that they control the preparation unit in such a way that the substituate is matched in its composition to a prescribed composition.

8. Blood treatment apparatus in accordance with one of the preceding claims, **characterized in that** the preparation unit is configured such that the conveying of bicarbonate takes place in dependence on the acetate concentration or citrate concentration of the solution prepared in the preparation unit.

9. Blood treatment apparatus in accordance with one of the preceding claims, **characterized in that** a conveying means is arranged in the dialyzate circuit for conveying dialysate, and **in that** the control or regulation unit is configured such that the conveying means is reduced with respect to its conveying rate or is stopped when the second means are active.

10. Blood treatment apparatus in accordance with one of the preceding claims, **characterized in that** the means are configured such that the flushing solution or the dialyzate or the substituate have a pH in the range between 7.3 and 7.7, preferably in the range between 7.35 and 7.7, and particular preferably in the range between 7.35 and 7.45.

11. A blood treatment apparatus in accordance with one of the preceding claims, **characterized in that** the preparation unit comprises a main line which is connected to a water port, in particular to an RO water port; and **in that** one or more concentrate lines which are in fluid communication with one or more concentrate reservoirs open into this main line.

## Revendications

1. Dispositif de traitement du sang comprenant un circuit extracorporel et un circuit de dialysat ainsi qu'un dialyseur, lequel comporte un compartiment côté sang et un compartiment côté dialysat séparé du compartiment côté sang par une membrane, le compartiment côté sang étant en communication fluidique avec le circuit extracorporel et le compartiment côté dialysat avec le circuit de dialysat, et comprenant une unité de préparation pour la fabrication en ligne d'une solution, **caractérisé en ce que** le dispositif de traitement du sang comporte des premiers moyens destinés à exécuter un mode d'amorçage et des deuxièmes moyens destinés à exécuter un mode de traitement initial, les premiers moyens étant conçus de manière à commander l'unité de préparation de telle façon qu'une solution de rinçage avec un pH ≥ 7,3 et de préférence ≥ 7,4 est fabriquée, et que le circuit extracorporel est rempli de cette solution de rinçage, et **en ce que** les deuxièmes moyens sont conçus de manière à commander l'unité de préparation de telle façon que du dialysat avec un pH ≥ 7,3 et de préférence ≥ 7,4 est fabriqué, et que le circuit de dialysat est rempli de ce dialysat, le dispositif de traitement du sang comportant une unité de commande ou de régulation, qui actionne les premiers moyens avant les deuxièmes moyens, l'unité de préparation comportant des moyens d'acheminement pour l'acheminement de bicarbonate et en particulier d'un concentré contenant du bicarbonate et les moyens d'acheminement étant conçus de manière à effectuer le réglage du pH au moyen de l'acheminement de bicarbonate, et le dispositif de traitement du sang comportant des troisièmes moyens pour exécuter un mode de traitement succédant au mode de traitement initial, les troisièmes moyens étant conçus de manière à commander l'unité de préparation de telle façon que la composition du dialysat est amenée à correspondre à une composition prescrite, la concentration en bicarbonate étant diminuée, et l'unité de commande ou de régulation étant conçue de telle manière que les troisièmes moyens sont actionnés après les deuxièmes moyens.

2. Dispositif de traitement du sang selon la revendication 1, **caractérisé en ce que** les premiers et les deuxièmes moyens sont conçus de manière à commander l'unité de préparation de manière identique, de sorte que les compositions de la solution de rinçage et du dialysat sont identiques dans le mode de traitement initial.

3. Dispositif de traitement du sang selon la revendication 1 ou 2, **caractérisé en ce que** les deuxièmes moyens ou d'autres moyens sont conçus de manière à commander l'unité de préparation de telle façon que du liquide de substitution avec un pH ≥ 7,3 et de préférence ≥ 7,4 est fabriqué, les deuxièmes moyens étant de préférence conçus de manière à commander l'unité de préparation pour la fabrication du dialysat et de liquide de substitution de manière identique, de telle sorte que les compositions du dialysat et du liquide de substitution sont identiques.

4. Dispositif de traitement du sang selon l'une des revendications précédentes, **caractérisé en ce que** les deuxièmes moyens sont réalisés de manière à être actionnés pendant un intervalle de temps prédéfini ou pendant une durée qui dépend d'au moins un paramètre.

5. Dispositif de traitement du sang selon la revendication 4, **caractérisé en ce que** le paramètre est la quantité du plasma extrait par le biais de la membrane du dialyseur ou un paramètre en corrélation avec celui-ci ou le coefficient d'ultrafiltration de la membrane du dialyseur.

6. Dispositif de traitement du sang selon l'une des revendications précédentes, **caractérisé en ce que** les premiers moyens sont conçus de telle manière que, outre le circuit extracorporel, le compartiment côté dialysat est également rempli de solution de rinçage.

7. Dispositif de traitement du sang selon l'une des revendications précédentes, **caractérisé en ce que** les troisièmes moyens sont conçus de manière à commander l'unité de préparation de telle façon que la composition du liquide de substitution est amenée à correspondre à une composition prescrite.

8. Dispositif de traitement du sang selon l'une des revendications précédentes, **caractérisé en ce que** l'unité de préparation est réalisée de telle manière que l'acheminement de bicarbonate est effectué en fonction de la concentration en acétate ou en citrate de la solution fabriquée dans l'unité de préparation.

9. Dispositif de traitement du sang selon l'une des revendications précédentes, **caractérisé en ce qu'**un moyen d'acheminement pour l'acheminement de dialysat est disposé dans le circuit de dialysat et **en ce que** l'unité de commande ou de régulation est conçue de telle manière que le débit d'acheminement du moyen d'acheminement est diminué ou stoppé, quand les deuxièmes moyens sont actifs.

10. Dispositif de traitement du sang selon l'une des revendications précédentes, **caractérisé en ce que** les moyens sont conçus de telle manière que la solution de rinçage ou le dialysat ou le liquide de substitution présente un pH compris entre 7,3 et 7,7, de préférence compris entre 7,35 et 7,7 et plus préférentiellement compris entre 7,35 et 7,45.

11. Dispositif de traitement du sang selon l'une des revendications précédentes, **caractérisé en ce que** l'unité de préparation comporte une ligne principale, qui est en liaison avec un raccordement d'eau, en particulier un raccordement d'eau traitée par osmose inverse, et **en ce qu'**une ou plusieurs lignes de concentré débouchent dans cette ligne principale, lesquelles sont en communication fluidique avec un ou plusieurs réservoirs de concentré.
